# EUROPEAN PATENT SPECIFICATION

(11) **EP 1 425 008 B1**
(45) Date of publication and mention of the grant of the patent: **04.10.2006**
(21) Application number: 02767352.4
(22) Date of filing: 09.08.2002
(51) Int. Cl.: A61K 31/353, A61K 31/195, A61P 25/14, A61P 25/16

(54) **USE OF SUBSTITUTED AMINOMETHYL CHROMANS IN THE TREATMENT OF SIDE EFFECTS OF NEUROLEPTICS**
VERWENDUNG VON AMINOMETHYL CHROMANEN ZUR BEHANDLUNG DER NEBENWIRKUNGEN VON NEUROLEPTIkA
UTILISATION D'AMINOMETHYLCHROMANNES SUBSTITUES POUR TRAITER LES EFFETS SECONDAIRES DES NEUROLEPTIQUES

(30) Priority: 12.09.2001 EP 01121910
(43) Date of publication of application: 09.06.2004
(73) Proprietor: Merck Patent GmbH, 64293 Darmstadt (DE)
(72) Inventor: BARTOSZYK, Gerd, 64331 Weiterstadt (DE); RUSS, Hermann, 64297 Darmstadt (DE); SEYFRIED, Christoph, 64342 Seeheim-Jugenheim (DE); BÖTTCHER, Henning, 64287 Darmstadt (DE); BOKEL, Heinz-Hermann, 64287 Darmstadt (DE); SCHMID-GROSSMANN, Uschi, 64625 Bensheim (DE)
(86) International application number: PCT/EP2002/008912
(87) International publication number: WO 2003/022269

(56) References cited:
- EP-A- 0 900 568
- DE-A- 10 101 917
- US-A- 4 983 400
- US-B1- 6 235 774
- "The Merck Manual - sixteenth edition" 1992 , MERCK RESEARCH LABORATORIES , RAHWAY, N.J. XP002223982 16 page 1491 -page 1503
- MEWSHAW R E ET AL: "New Generation Dopaminergic Agents. 1. Discovery of a Novel Scaffold Which Embraces the D2 Agonist Pharmacophore. Structure-Activity Relationship of a Series of 2-(Aminomethyl)chromans" JOURNAL OF MEDICINAL CHEMISTRY, AMERICAN CHEMICAL SOCIETY. WASHINGTON, US, vol. 40, no. 26, 1997, pages 4235-4256, XP002155829 ISSN: 0022-2623

## Description

The present invention relates to the use of substituted aminomethyl chromans of formula I 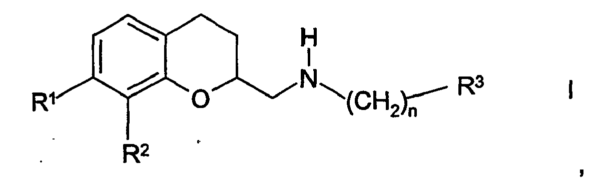 in which
R¹ represents hydrogen,
R² represents hydrogen, hydroxyl or a radical of the formula -OCH₃, -OCH₂CH₃, -OCH(CH₃)₂ or -CH₂C(CH₃)₂-Cl, or
R¹ and R² together form a radical of the formula 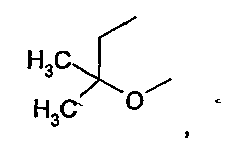
R³ represents cyclopentyl, cyclohexyl, cycloheptyl, or the following radical, designated as o-benzenesulphimidyl: 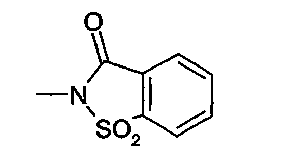
and
n is selected from 1, 2, 3, 4 or 5,
and their optical isomers and pharmaceutically acceptable salts,
in particular (-)-(R)-2-{4-[[3,4-dihydro-2H-benzopyran-2-yl)-methyl]amino]butyl}-1,2-benzoisothiazol-3-(2H)-on-1,1-dioxide or a physiologically acceptable salt thereof, for the manufacture of a medicament for the treatment of adverse effects of anti-Parkinsonian drugs in extrapyramidal movement disorders and/or for the manufacture of a medicament for the treatment of extrapyramidal symptoms (EPS) induced by neuroleptics.

EP-A-0 352 613, EP-A-0 540 914 and EP-A-0 749 970 describe aminomethyl chroman derivatives which are suitable for the prophylaxis, neuroprotection and treatment of formation of cerebral infarcts (cerebral apoplexy) such as stroke and cerebral ischaemia.
The therapeutic efficacy of the compounds described in this document is unknown.
The principle of the preparation of the aminomethyl chromans to be used according to the invention is disclosed in EP-A-0 352 613, EP-A-0 540 914 and EP-A-0 749 970.

Preferred compounds in the context of the invention are those of the general formula I 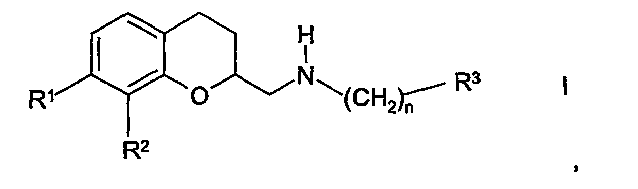 where
R¹ represents hydrogen,
R² represents hydrogen, hydroxyl or a radical of the formula -OCH₃, -OCH₂CH₃, -OCH(CH₃)₂ or -CH₂C(CH₃)₂-Cl, or
R¹ and R² together form a radical of the formula 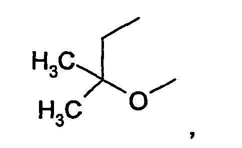
R³ represents o-benzenesulphimidyl,
n=3 or 4;
and aminomethyl chromans of the general formula I 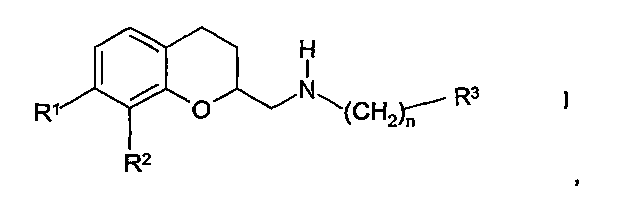 in which
R¹ represents hydrogen,
R² represents hydrogen, hydroxyl or a radical of the formula -OCH₃ or -OCH(CH₃)₂, or
R¹ and R² together form a radical of the formula 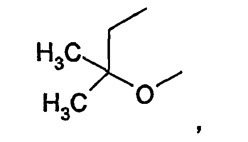
n = 1 and
R³ represents cyclohexyl or cycloheptyl.

A particularly preferred compound, (-)-(R)-2-{4-[[3,4-dihydro-2H-benzopyran-2-yl)-methyl]amino]butyl}-1,2-benzoisothiazol-3-(2H)-on-1,1-dioxide is known from EP-A-0 352 613, example 92. The compound which is referred to herein is described in the patent as 5-HT_{1A} receptor agonist. Therefore, the use of (-)-(R)-2-{4-[[3,4-dihydro-2H-benzopyran-2-yl)-methyl]amino]butyl}-1,2-benzoisothiazol-3-(2H)-on-1,1-dioxide or a pharmaceutically acceptable salt thereof for the manufacture of a medicament for prophylaxis and control of the sequelae of cerebral infarction (apoplexia cerebri) such as stroke and cerebral ischaemia, for prophylaxis and control of cerebral disorders, e.g. migraine, the treatment of anxiety, tension and depression states, sexual dysfunctions caused by the central nervours system, for disturbances in sleep or absorption of food is disclosed.
In the context of the present invention, the aminomethyl chroman compounds of formula I can be present in various stereoisomeric forms, i.e. in the form of their (+) or (-) enantiomers or as a mixture of these enantiomers (racemate). For the separation of the racemates into the enantiomeric forms, reference is made to the relevant, known specialist literature.

In the context of the present invention, the physiologically acceptable salts can also be employed. Physiologically acceptable salts of the substituted 2-aminomehyl chromans of formula I can be salts of the compounds according to the invention with suitable organic or inorganic acids, in particular mineral acids, carboxylic acids or sulphonic acids. Particularly preferred salts are, for example, those with hydrochloric acid, hydrobromic acid, sulphuric acid, phosphoric acid, methanesulphonic acid, ethanesulphonic acid, toluenesulphonic acid, benzenesulphonic acid, naphthalene-disulphonic acid, acetic acid, propionic acid, lactic acid, tartaric acid, citric aid, fumaric acid, maleic acid or benzoic acid.

The invention had the object of providing novel uses for substituted aminomethyl chromans of formula I, in particular of (-)-(R)-2-{4-[[3,4-dihydro-2H-benzopyran-2-yl)-methyl]amino]butyl}-1,2-benzoisothiazol-3-(2H)-on-1,1-dioxide and its physiologically acceptable salts.

It has been found that the substituted aminomethyl chromans of formula I, in particular (-)-(R)-2-{4-[[3,4-dihydro-2H-benzopyran-2-yl)-methyl]amino]butyl}-1,2-benzoisothiazol-3-(2H)-on-1,1-dioxide or physiologically acceptable salts also have therapeutic activity against extrapyramidal movement disorders such as dyskinetic, choreatic, or dystonic syndromes, tremor, Gilles de la Torette syndrome, ballism, myoclonus, restless legs syndrome or Wilson's disease, as well as extrapyramidal motoric disturbances [synonymous extrapyramidal symptoms (EPS)] induced by neuroleptics.

Additionally it has been found that substituted aminomethyl chromanes of formula I, in particular (-)-(R)-2-{4-[[3,4-dihydro-2H-benzopyran-2-yl)-methyl]amino]butyl}-1,2-benzoisothiazol-3-(2H)-on-1,1-dioxide or physiologically acceptable salts have therapeutic activity against adverse effects of anti-Parkinsonian drugs in extramyramidal movement disorders, in particular against dopaminomimetic adverse effects of anti-Parkinsonian drugs in idiopathic Parkinson's disease or Parkinson syndromes.

It has been found that (-)-(R)-2-{4-[[3,4-dihydro-2H-benzopyran-2-yl)-methyl]amino]butyl}-1,2-benzoisothiazol-3-(2H)-on-1,1-dioxide or physiologically acceptable salts excerts an extraordinary potency in reversing catalepsy. Extrapyramidal motor side effects in e.g. rodents are measured by the ability of a drug to induce catalepsy. Catalepsy is defined as a state where an animal continues to remain in an unnormal (nonphysiological 'uncomfortable') posture for a long time (*e.g*.: M.E. Stanley and S.D. Glick, Neuropharmacology, 1996; 15: 393-394; C.J.E. Niemegeers and P. Janssen, Life Sci., 1979, 201-2216). For example, if a hindpaw of a rat is placed on an elevated level, e.g. a platform elevated 3 cm above ground level, a normal rat immediately withdraws the hindpaw from the platform to the ground level. A cataleptic rat remains in this unnatural posture even for minutes.

Beneficial effects on the extrapyramidal motoric system have previously been described for other drugs with 5-HT_{1A} agonistic action. Buspirone for example, which is an anxiolytic drug by nature, exhibits moderate anti-dyskinetic properties in advanced Parkinson patients (B. Kleedorfer et al., J Neurol Neurosurg Psychiatry, 1991, **54**: 376-377; V. Bonifati et al., Clin Neuropharmacol, 1994, **17**: 73-82). The main mechanism of action is obviously via stimulation of 5-HT_{1A} receptors of the raphe nigral and raphe striatal pathways.
In rats, the oral ED₅₀ value (i.e. the calculated dose to reverse catalepsy by 50%) for (-)-(R)-2-{4-[[3,4-dihydro-2H-benzopyran-2-yl)-methyl]amino]butyl}-1,2-benzoisothiazol-3-(2H)-on-1,1-dioxide monohydrochloride is 0,6 mg/kg which unexpectedly is 30-100 times more potent compared to other 5-HT_{1A} agonists such as ipsapirone (ED₅₀ 23 mg/kg), buspirone (ED₅₀ 30 mg/kg), gepirone (ED₅₀ 16 mg/kg) or tandosirone (ED₅₀ 60 mg/kg).

Therefore, the present invention relates to the use of substituted aminomethyl chromans of formula I 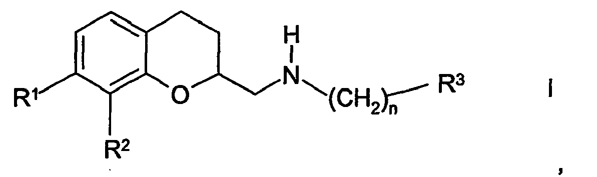 in which
R¹ represents hydrogen,
R² represents hydrogen, hydroxyl or a radical of the formula -OCH₃, -OCH₂CH₃, -OCH(CH₃)₂ or -CH₂C(CH₃)₂-Cl, or
R¹ and R² together form a radical of the formula 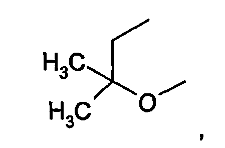
R³ represents cyclopentyl, cyclohexyl, cycloheptyl, or the following radical, designated as o-benzenesulphimidyl: 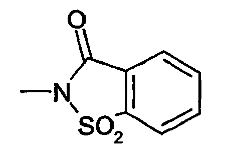
and
n is selected from 1, 2, 3, 4 or 5,
and their optical isomers and pharmaceutically acceptable salts for the manufacture of a medicament for the treatment of extrapyramidal movement disorders.

A particularly preferred compound of formula I is (-)-(R)-2-{4-[[3,4-dihydro-2H-benzopyran-2-yl)-methyl]amino]butyl}-1,2-benzoisothiazol-3-(2H)-on-1,1-dioxide or a physiologically acceptable salt thereof.

Therefore, the invention relates to the use of (-)-(R)-2-{4-[[3,4-dihydro-2H-benzopyran-2-yl)-methyl]amino]butyl}-1,2-benzoisothiazol-3-(2H)-on-1,1-dioxide or a pharmacologically acceptable salt for the manufacture of a medicament for the treatment of extrapyramidal movement disorders.

A preferred salt of (-)-(R)-2-{4-[[3,4-dihydro-2H-benzopyran-2-yl)-methyl]amino]butyl}-1,2-benzoisothiazol-3-(2H)-on-1,1-dioxide is (-)-(R)-2-{4-[[3,4-dihydro-2H-benzopyran-2-yl)-methyl]amino]butyl}-1,2-benzoisothiazol-3-(2H)-on-1,1-dioxide monohydrochloride.

Therefore, the invention relates to the use for the manufacture of a medicament for the treatment of extrapyramidal movement disorders in which the pharmacologically acceptable salt is (-)-(R)-2-{4-[[3,4-dihydro-2H-benzopyran-2-yl)-methyl]amino]butyl}-1,2-benzoisothiazol-3-(2H)-on-1,1-dioxide monohydrochloride.

Additionally, the invention relates to the use of a pharmaceutical composition containing at least one compound of (-)-(R)-2-{4-[[3,4-dihydro-2H-benzopyran-2-yl)-methyl]amino]butyl}-1,2-benzoisothiazol-3-(2H)-on-1,1-dioxide or one of its biocompatible salts for the treatment of extrapyramidal movement disorders induced by neuroleptics.

(-)-(R)-2-{4-[[3,4-dihydro-2H-benzopyran-2-yl)-methyl]amino]butyl}-1,2-benzoisothiazol-3-(2H)-on-1,1-dioxide or a physiologically acceptable salt, useful for the treatment of extrapyramidal movement disorders, in particular for the treatment of dyskinetic, choreatic or dystonic syndromes, extrapyramidal motoric adverse effects of neuroleptics, tremor, Gilles de la Tourette syndrome, ballism, myoclonus, restless legs syndrome or Wilson's disease and/or useful for the treatment of adverse effects in idiopathic Parkinson's disease or Parkinson syndromes including medicinal compositions as defined below, is preferably administered in doses from 0.01 to 100 mg, preferentially between approximately 0,1 and 10 mg. The composition may be administered in daily doses. The specific dose for each patient depends on all sorts of factors, e.g. on the activity of the specific compound employed, on the age, body weight, general state of health, on sex, diet, time and route of administration, on the excretion rate, pharmaceutical substance combination and on the severity of the particular disorder to which the therapy relates. Oral administration is preferred, but also parenteral routes of administration (e.g. intravenous or transdermal) can be utilized.

Anti-Parkinsonian drugs are conventional drugs such as I-dopa (levodopa) and I-dopa combined with benserazide or carbidopa, dopamine agonists such as bromocriptine, apomorphine, cabergoline, pramipexol, ropinirol, pergolide, dihydro-α-ergocriptine or lisuride plus all drugs acting via stimulation of dopamine receptors, inhibitors of catechol-O-methyl transferase (COMT) such as entacapone or tolcapone, inhibitors of monoamine oxidase (MAO) such as selegiline and antagonists of N-methyl-D-aspartate (NMDA) receptors such as amantadine or budipine.

Adverse effects of said anti-Parkinsonian drugs are all types of dyskinesias, such as choreic, dystonic, ballistic and myoclonic dyskinesia, as well as motor (response) fluctuations or psychotic states.

Therefore, the present invention relates to the use of substituted aminomethyl chromans of formula I 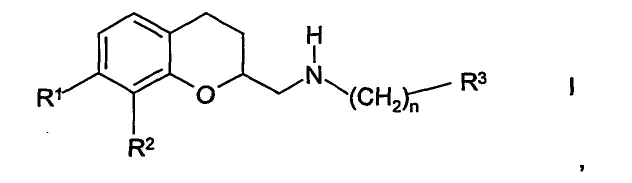 in which
R¹ represents hydrogen,
R² represents hydrogen, hydroxyl or a radical of the formula -OCH₃, -OCH₂CH₃, -OCH(CH₃)₂ or -CH₂C(CH₃)₂-Cf, or
R¹ and R² together form a radical of the formula 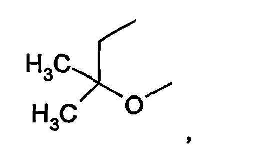
R³ represents cyclopentyl, cyclohexyl, cycloheptyl, or the following radical, designated as o-benzenesulphimidyl: 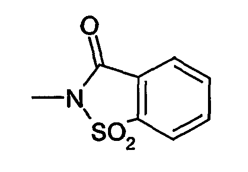
and
n is selected from 1, 2, 3, 4 or 5,
and their optical isomers and pharmaceutically acceptable salts for the manufacture of a medicament for the treatment of adverse effects of anti-Parkinsonian drugs in idiopathic Parkinson's disease.

A particularly preferred compound of formula I is (-)-(R)-2-{4-[[3,4-dihydro-2H-benzopyran-2-yl)-methyl]amino]butyt}-1,2-benzoisothiazol-3-(2H)-on-1,1-dioxide or a physiologically acceptable salt thereof.

Therefore, the invention relates to the use of (-)-(R)-2-{4-[[3,4-dihydro-2H-benzopyran-2-yl)-methyl]amino]butyl}-1,2-benzoisothiazol-3-(2H)-on-1,1-dioxide or a pharmacologically acceptable salt for the manufacture of a medicament for the treatment of adverse effects of anti-Parkinsonian drugs in idiopathic Parkinson's disease.

Treatment of adverse effects of conventional anti-Parkinsonian drugs as defined above are determined in a modification of the animal model of the Parkinsonian cynomolgus monkey according to P.J. Blanchet et al., Exp. Neurology 1998; 153: 214-222. Monkeys render parkinsonian by repeated injections of 1-methyl-4-phenyl-1,2,3,6-tetrahydropyridine (MPTP). The Parkinsonian monkeys are chronically treated with the standard I-dopa therapy according to P.J. Blanchet *et al.,* Mov. Disord., 1998; **13**: 798-802. Longterm treatment with I-dopa induces extrapyramidal motor side effects and psychotic states which are both qualitatively and quantitatively, assessed by the Abnormal involuntary Movement Scale (P.J. Blanchet *et al.,* Mov. Disord. 1998; **13:** 798-802) for different body parts (face, neck, trunk, each limb) and by rating for psychotic states by observing the monkey's attention, reactivity and mobility. (-)-(R)-2-{4-[[3,4-dihydro-2H-benzopyran-2-yl)-methyl]amino]butyl}-1,2-benzoisothiazol-3-(2H)-on-1,1-dioxide reduced overall choreiform dyskinesias and dystonic dyskinesias as well as psychotic states.

A typical study to investigate the efficacy of the compounds according to the invention for adverse effects in Parkinson's disease is described in the following. 40 patients of either sex with advanced idiopathic Parkinson's disease complicated by "peak-dose" dyskinesia participate in a double-blind study. The main inclusion criteria are Hoehn & Yahr stage ≥ 2.5 (lit.: Hoehn H.M. et al, Neurology 1967; 17: 427-442), aged 40-75 years, symptom duration of at least 5 years, and a I-dopa treatment duration of at least 3 years. (-)-(R)-2-{4-[[3,4-dihydro-2H-benzopyran-2-yl)-methyl]amino]butyl}-1,2-benzoisothiazol-3-(2H)-on-1,1-dioxide monohydrochloride or placebo is administered as "add on" to the conventional Parkinson treatment, which is maintained unchanged during the whole study. The dose of blinded medication is titrated over a period of 3 weeks in a range from 2.5 to 10 mg b.i.d. Then the medication is kept constant for 3 weeks. Before the start of titration and at the end of the treatment period the patients fill a diary card in 30 min. inervalls for 48 hours. The diary card differentiates 5 different states: (1) "on" without dyskinesia, (2) "on" with troublesome dyskinesia, (3) "on" with non-troublesome dyskinesia, (4) "off' time, and (5) time asleep (Hauser RA et al., Clin. Neuropharmacol., 2000, 23, 75-81). The primary outcome variable of the protocol is the change in "on" time with troublesome dyskinesia. The statistical analysis of the diary data demonstrates a significant reduction in "on" time with troublesome dyskinesia under treatment with (-)-(R)-2-{4-[[3,4-dihydro-2H-benzopyran-2-yl)-methyl]amino]butyl)-1,2-benzoisothiazol-3-(2H)-on-1,1-dioxide monohydrochloride while the "on" time without dyskinesia significantly increase. The other parameters are not changed.

A preferred salt of (-)-(R)-2-{4-[[3,4-dihydro-2H-benzopyran-2-yl)-methyl]amino]butyl}-1,2-benzoisothiazol-3-(2H)-on-1,1-dioxide is (-)-(R)-2-{4-[[3,4-dihydro-2H-benzopyran-2-yl)-methyl]amino]butyl}-1,2-benzoisothiazol-3-(2H)-on-1,1-dioxide monohydrochloride.

Therefore, the invention relates to the use for the manufacture of a medicament for the treatment of adverse effects of anti-Parkinsonian drugs in idiopathic Parkinson's disease in which the pharmacologically acceptable salt is (-)-(R)-2-{4-[[3,4-dihydro-2H-benzopyran-2-yl)-methyl]amino]butyl}-1,2-benzoisothiazol-3-(2H)-on-1,1-dioxide monohydrochloride.

Additionally, the invention relates to the use of a pharmaceutical composition containing at least one compound of (-)-(R)-2-{4-[[3,4-dihydro-2H-benzopyran-2-yl)-methyl]amino]butyl}-1,2-benzoisothiazol-3-(2H)-on-1,1-dioxide or one of its biocompatible salts for the treatment of adverse effects of anti-Parkinsonian drugs in idiopathic Parkinson's disease.

The limiting factor of Parkinson treatment with I-dopa and/or dopamine agonists is often the occurence of psychosis or dyskinesia and other motor fluctuations.

It has been found that (-)-(R)-2-{4-[[3,4-dihydro-2H-benzopyran-2-yl)-methyl]amino]butyl}-1 ,2-benzoisothiazol-3-(2H)-on-1, -dioxide or a physiologically acceptable salt thereof enhance the anti-Parkinsonian effect of anti-Parkinsonian drugs as defined above without inducing extrapyramidal side effects.

Therefore, the add-on therapy with (-)-(R)-2-{4-[[3,4-dihydro-2H-benzopyran-2-yl)-methyl]amino]butyl)-1,2-benzoisothiazol-3-(2H)-on-1,1-dioxide or a physiologically acceptable salt thereof, in particular of (-)-(R)-2-{4-[[3,4-dihydro-2H-benzopyran-2-yl)-methyl]amino]butyl}-1,2-benzoisothiazol-3-(2H)-on-1,1-dioxide monohydrochloride, now opens the possibility to increase the doses of I-dopa and/or dopamine agonists and/or all other anti-Parkinsonian drugs as defined above in order to counteract periods of insufficient motility ("off" phases) without provoking the above mentioned side effects. That represents an entirely novel approach in the treatment of Parkinson's disease leading to a significant benefit for the patients.

Thus, the invention relates to a pharmaceutical composition comprising, as active principles, (i) (-)-(R)-2-{4-[[3,4-dihydro-2H-benzopyran-2-yl)-methyl]amino]butyl}-1,2-benzoisothiazol-3-(2H)-on-1,1-dioxide or a physiologically acceptable salt thereof, and (ii) at least one anti-Parkinsonian drug, in combination with one or more pharmaceutically acceptable excipients.

Particularly, the invention relates to a pharmaceutical composition comprising, as active principles, (i) (-)-(R)-2-{4-[[3,4-dihydro-2H-benzopyran-2-yl)-methyl]amino]butyl}-1,2-benzoisothiazol-3-(2H)-on-1,1-dioxide monohydrochloride, and (ii) I-dopa or I-dopa combined with benserazide or carbidopa, in combination with one or more pharmaceutically acceptable excipients.

The ratios of the respective amounts of (-)-(R)-2-{4-[[3,4-dihydro-2H-benzopyran-2-yl)-methyl]amino]butyl}-1,2-benzoisothiazol-3-(2H)-on-1,1-dioxide or one of its physiologically acceptable salts and of the conventional anti-Parkinsonian drug thus vary in consequences. Preferably, the weight ratio of (-)-(R)-2-{4-[[3,4-dihydro-2H-benzopyran-2-yl)-methyl]amino]butyl}-1,2-benzoisothiazol-3-(2H)-on-1,1-dioxide or one of its physiologically acceptable salts to the conventional anti-Parkinsonian drug ranges from 1:1 to 1:100, preferably from 1:10 to 1:90 and better still from 1:40 to 1:60.

According to the invention, the term "medicinal combination" is intended to refer either to a pharmaceutical composition as defined above, in which the two active principles or compounds are the essential constituents of the same composition, or to a kit comprising two separate compositions, the first comprising (-)-(R)-2-{4-[[3,4-dihydro-2H-benzopyran-2-yl)-methyl]amino]butyl}-1,2-benzoisothiazol-3-(2H)-on-1,1-dioxide or one of its physiologically acceptable salts as sole active principle, and the second comprising at least one anti-Parkinsonian drug as active compound.

When the medicinal combination is in the form of a kit, the administration of the two compositions constituting this kit, although carried out separately, is simultaneous for a combined therapy. It is preferred to use (-)-(R)-2-{4-[[3,4-dihydro-2H-benzopyran-2-yl)-methyl]amino]butyl}-1,2-benzoisothiazol-3-(2H)-on-1,1-dioxide in the form of the monohydrochloride.

Adverse effects of anti-Parkinsonian drugs as defined above are additionally known in particular in Parkinson syndromes.

Parkinson syndromes are e.g. multiple system atrophies (MSA), Steele-Richardson-Olszewski syndrome (= progressive supranuclear palsy), cortico-basal degeneration, olivo-ponto cerebellar atrophy or Shy Drager syndrome.

Therefore, the invention relates to the use of substituted aminomethyl chromans of formula I 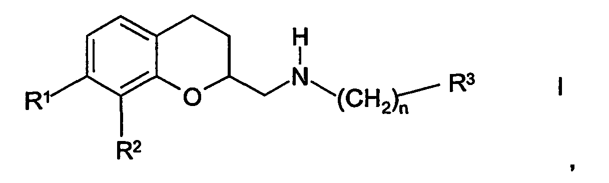 in which
R¹ represents hydrogen,
R² represents hydrogen, hydroxyl or a radical of the formula -OCH₃, -OCH₂CH₃, -OCH(CH₃)₂ or -CH₂C(CH₃)₂-Cl, or
R¹ and R² together form a radical of the formula 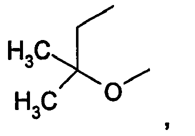
R³ represents cyclopentyl, cyclohexyl, cycloheptyl, or the following radical, designated as o-benzenesulphimidyl: 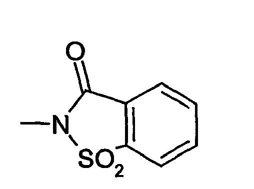
and
n is selected from 1, 2, 3, 4 or 5,
and their optical isomers and pharmaceutically acceptable salts for the manufacture of a medicament for the treatment of adverse effects of anti-Parkinsonian drugs in Parkinson syndromes.

A particularly preferred compound of formula I is (-)-(R)-2-{4-[[3,4-dihydro-2H-benzopyran-2-yl)-methyl]amino]butyl}-1,2-benzoisothiazol-3-(2H)-on-1,1-dioxide or a physiologically acceptable salt thereof.

Therefore, the invention relates to the use of (-)-(R)-2-{4-[[3,4-dihydro-2H-benzopyran-2-yl)-methyl]amino]butyl}-1,2-benzoisothiazol-3-(2H)-on-1,1-dioxide or a pharmacologically acceptable salt for the manufacture of a medicament for the treatment of adverse effects of anti-Parkinsonian drugs in Parkinson syndromes.

A typical animal model is the reserpinized rat or mouse (*e.g.* M.S. Starr and B.S. Starr, J. Neural Transm. - Park. Dis. Dement. Sect., 1994; 7: 133-142; M. Gossel et al., J. Neural Transm. - Park. Dis. Dement. Sect., 1995; **10**: 27-39; N.R. Hughes *et al.*, Mov. Disord., 1998; 13: 228-233). Reserpine is a potent depleter of monoamines and produces nearly complete akinesia in both species. Prominent 24 h after application, the distance travelled and the time active is nearly zero as measured in conventional activity meters. (-)-(R)-2-{4-[[3,4-dihydro-2H-benzopyran-2-yl)-methyl]amino]butyl}-1,2-benzoisothiazol-3-(2H)-on-1,1-dioxide or a pharmaceutically acceptable salt thereof dose-dependently reduced akinesia, *i.e.* restored distance travelled and time active to about the level of normal animals.

Another more recent animal model is the striatonigral degeneration approach in the rat according to G.K. Wenning *et al.,* J. Neural Transm. Suppl., 1999; **55:** 103-113. Rats receive an unilateral injection of 6-hydroxydopamine into the left medial forebrain bundle followed by an injection of quinolinic acid into the ipsilateral striatum inducing nigrostriatal degeneration. The degeneration results in turning behavior to a challenge with dopaminomimetics such as apomorphine or amphetamine. Turning behavior is measured by an automated recorder. Turning behavior induced by apomorphine or amphetamine was dose-dependently antagonized by (-)-(R)-2-{4-[[3,4-dihydro-2H-benzopyran-2-yl)-methyl]amino]butyl}-1,2-benzoisothiazol-3-(2H)-on-1,1-dioxide or a pharmaceutically acceptable salt thereof.

Multiple system atrophy (MSA) is due to an expansive neurodegeneration in the extrapyramidal and autonomic nervous system which leads to an akinetic Parkinsonian syndrome with vegetative disturbances. In contrast to idiopathic Parkinson's disease the density of central dopamine receptors is markedly decreased and therefore, MSA patients poorly respond to dopaminergic drugs. Since (-)-(R)-2-{4-[[3,4-dihydro-2H-benzopyran-2-yl)-methyl]amino]butyl}-1,2-benzoisothiazol-3-(2H)-on-1,1-dioxide or a pharmaceutically acceptable salt thereof act predominantly via serotonin receptors on the extrapyramidal system, they are able to improve the motor performance in these otherwise mostly untreatable patients.

A typical study to investigate the efficacy of the compounds according to the invention in MSA patients encompasses 30 patients of either sex with a symptom duration of at least 5 years and a significant reduction of central dopamine receptors in positron emission tomography (PET) scan. The study design is similar to that described above for Parkinson's disease. (-)-(R)-2-{4-[[3,4-dihydro-2H-benzopyran-2-yl)-methyl]amino]butyl}-1,2-benzoisothiazol-3-(2H)-on-1,1-dioxide monohydrochloride or placebo is titrated as "add on" to the conventional treatment (dose range 1 to 20 mg t.i.d.). After a double-bllind dose-finding phase of 3 weeks during which the individual dose is identified for each patient on the basis of tolerability and efficacy, the dose is maintained unchanged for 3 additional weeks. Before the start of titration and at the end of the treatment period a complete UPDRS assessment is performed in each patient (primary outcome measure). Statistical analysis of UPDRS demonstrates a significant clinical improvement of Parkinson symptoms under treatment with (-)-(R)-2-{4-[[3,4-dihydro-2H-benzopyran-2-yl)-methyl]amino]butyl}-1,2-benzoisothiazol-3-(2H)-on-1,1-dioxide monohydrochloride.

A preferred salt of (-)-(R)-2-{4-[[3,4-dihydro-2H-benzopyran-2-yl)-methyl]amino]butyl}-1,2-benzoisothiazol-3-(2H)-on-1,1 -dioxide is (-)-(R)-2-{4-[[3,4-dihydro-2H-benzopyran-2-yl)-methyl]amino]butyl}-1,2-benzoisothiazol-3-(2H)-on-1,1-dioxide monohydrochloride.

Therefore, the invention relates to the use for the manufacture of a medicament for the treatment of adverse effects of anti-Parkinsonian drugs in Parkinson syndromes in which the pharmacologically acceptable salt is (-)-(R)-2-{4-[[3,4-dihydro-2H-benzopyran-2-yl)-methyl]amino]butyl}-1,2-benzoisothiazol-3-(2H)-on-1,1-dioxide monohydrochloride.

Additionally, the invention relates to the use of a pharmaceutical composition containing at least one compound of (-)-(R)-2-{4-[[3,4-dihydro-2H-benzopyran-2-yl)-methyl]amino]butyl}-1,2-benzoisothiazol-3-(2H)-on-1,1-dioxide or one of its biocompatible salts for the treatment of adverse effects of anti-Parkinsonian drugs in Parkinson syndromes.

The present invention relates furthermore to the use of substituted aminomethyl chromans of formula I 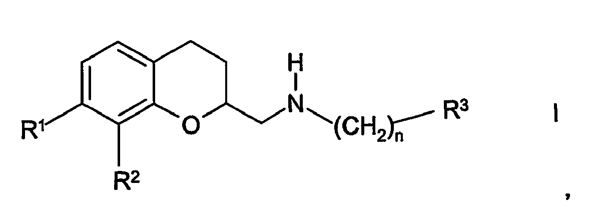 in which
R¹ represents hydrogen,
R² represents hydrogen, hydroxyl or a radical of the formula -OCH₃, -OCH₂CH₃, -OCH(CH₃)₂ or -CH₂C(CH₃)₂-Cl, or
R¹ and R² together form a radical of the formula 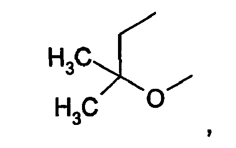
R³ represents cyclopentyl, cyclohexyl, cycloheptyl, or the following radical, designated as o-benzenesulphimidyl: 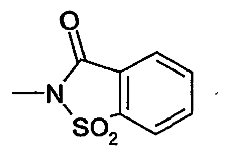
and
n is selected from 1, 2, 3, 4 or 5,
and their optical isomers and pharmaceutically acceptable salts for the manufacture of a medicament for the treatment of dyskinetic and/or choreatic syndromes.

A particularly preferred compound of formula I is (-)-(R)-2-{4-[[3,4-dihydro-2H-benzopyran-2-yl)-methyl]amino]butyl}-1,2-benzoisothiazol-3-(2H)-on-1,1-dioxide or a physiologically acceptable salt thereof.

Therefore, the invention relates to the use of (-)-(R)-2-{4-[[3,4-dihydro-2H-benzopyran-2-yl)-methyl]amino]butyl}-1,2-benzoisothiazol-3-(2H)-on-1,1-dioxide or a pharmacologically acceptable salt for the manufacture of a medicament for the treatment of dyskinetic and/or choreatic syndromes.

Dyskinetic and/or choreatic syndromes are e.g. Huntington's disease, minor chorea or chorea of pregnancy. (-)-(R)-2-{4-[[3,4-dihydro-2H-benzopyran-2-yl)-methyl]amino]butyl}-1,2-benzoisothiazol-3-(2H)-on-1,1-dioxide or a physiologically acceptable salt thereof are in particular useful for the treatment of Huntington's disease.

A typical animal model is the systemic 3-nitropropionic acid (3-NP) model in rats according to C.V. Borlongan *et al.,* Brain Res., 1995; **697**: 254-257. Rats are treated with injections of the selective striatal neurotoxin 3-NP i.p. every fourth day (C.V. Borlongan *et al.*, Brain Res. Protocols, 1997; 1: 253-257). After two injections of 3-NP, rats display nocturnal hyperactivity reflecting symptoms of early Huntington's disease, whereas rats treated with four injections of 3-NP display nocturnal akinesia (hypoactivity) reflecting symptoms of late Huntington's disease. Nocturnal activity is automatically measured in conventional acitivity cages by infrared beams. (-)-(R)-2-{4-[[3,4-dihydro-2H-benzopyran-2-yl)-methyl]amino]butyl}-1,2-benzoisothiazol-3-(2H)-on-1,1-dioxide or a pharmaceutical acceptable salt thereof reduced both the nocturnal hyperactivity and akinesia.

A typical trial to establish the effect of the compounds according to the invention on chorea, voluntary motor performance, and functional disability in patients with Huntington's disease encompasses 32 genetically diagnosed patients. (-)-(R)-2-{4-[[3,4-dihydro-2H-benzopyran-2-yl)-methyl]amino]butyl}-1,2-benzoisothiazol-3-(2H)-on-1,1-dioxide monohydrochloride or placebo is administered as "add on" to the conventional treatment, which is maintained unchanged during the whole study. The dose of blinded medication is titrated over a period of 3 weeks in a range from 2.5 to 20 mg b.i.d. Then the medication is held constant for 1 week. Assessments are performed in the week before and at the last day of the trial. Chorea is scored using the abnormal involuntary movement scale (AIMS, W. Guy, in: ECDEU assessment manual. Rockville MD: US dept. of health, education and welfare, 1976: 534-537), the unified Huntington's disease rating scale (UHDRS, Huntington study group, 1996, Movement Disord, **11:** 136-42), and judgement of video recordings. Voluntary motor performance is assessed using the UHDRS motor scale. Patients and their partners complete a questionnaire regarding functional disability. Statistical analysis demonstrates significant improvement of voluntary and involuntary motor performance in Huntington patients under treatment with (-)-(R)-2-{4-[[3,4-dihydro-2H-benzopyran-2-yl)-methyl]amino]butyl}-1,2-benzoisothiazol-3-(2H)-on-1,1-dioxide or a physiologically acceptable salt thereof.

A preferred salt of (-)-(R)-2-{4-[[3,4-dihydro-2H-benzopyran-2-yl)-methyl]amino]butyl}-1,2-benzoisothiazol-3-(2H)-on-1,1-dioxide is (-)-(R)-2-{4-[[3,4-dihydro-2H-benzopyran-2-yl)-methyl]amino]butyl}-1,2-benzoisothiazol-3-(2H)-on-1,1-dioxide monohydrochloride.

Therefore, the invention relates to the use for the manufacture of a medicament for the treatment of dyskinetic and/or choreatic syndromes, in particular for the treatment of Huntington's disease, in which the pharmacologically acceptable salt is (-)-(R)-2-{4-[[3,4-dihydro-2H-benzopyran-2-yl)-methyl]amino]butyl}-1,2-benzoisothiazol-3-(2H)-on-1,1-dioxide monohydrochloride.

Additionally, the invention relates to the use of a pharmaceutical composition containing at least one compound of (-)-(R)-2-{4-[[3,4-dihydro-2H-benzopyran-2-yl)-methyl]amino]butyl}-1,2-benzoisothiazol-3-(2H)-on-1,1-dioxide or one of its biocompatible salts for the treatment of dyskinetic and/or choreatic syndromes.

The present invention relates to the use of substituted aminomethyl chromans of formula I 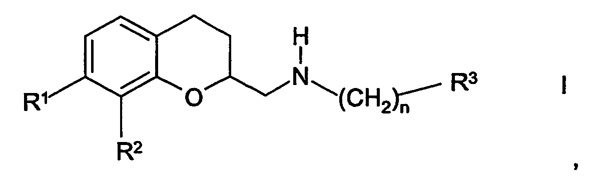 in which
R¹ represents hydrogen,
R² represents hydrogen, hydroxyl or a radical of the formula -OCH₃, -OCH₂CH₃, -OCH(CH₃)₂ or -CH₂C(CH₃)₂-Cl, or
R¹ and R² together form a radical of the formula 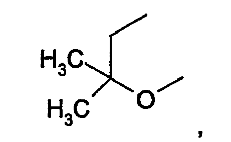
R³ represents cyclopentyl, cyclohexyl, cycloheptyl, or the following radical, designated as o-benzenesulphimidyl: 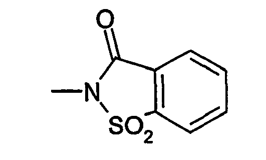
and
n is selected from 1, 2, 3, 4 or 5,
and their optical isomers and pharmaceutically acceptable salts for the manufacture of a medicament for the treatment of dystonic syndromes.

A particularly preferred compound of formula I is (-)-(R)-2-{4-[[3,4-dihydro-2H-benzopyran-2-yl)-methyl]amino]butyl}-1,2-benzoisothiazol-3-(2H)-on-1,1-dioxide or a physiologically acceptable salt thereof.

Therefore, the invention relates to the use of (-)-(R)-2-{4-[[3,4-dihydro-2H-benzopyran-2-yl)-methyl]amino]butyl}-1,2-benzoisothiazol-3-(2H)-on-1,1-dioxide or a pharmacologically acceptable salt for the manufacture of a medicament for the treatment of dystonic syndromes.

Dystonic syndromes are e.g. spasmalic torticollis, writer's cramp, blepharospasm, Meige syndrome or dopasensitive dystonia. (-)(R)-2-{4-[[3,4-dihydro-2H-benzopyran-2-yl)-methyl]amino]butyl}-1,2-benzoisothiazol-3-(2H)-on-1,1-dioxide or a physiologically acceptable salt thereof is in particular useful for the treatment of spasmalic torticollis and/or blepharospasm.

A typical animal model is the mutant dystonic hamster according to A. Richter and W. Löscher, Prog. Neurobiol. 1998; **54**: 633-677. In this genetically dystonic hamsters, dystonic attacks are provoked by taking the animal from the home cage and placing it on a balance. The dystonic syndrome consists of a sequence of abnormal movements, and the severity of the single symptoms is rated by a scoring system. (-)-(R)-2-{4-[[3,4-dihydro-2H-benzopyran-2-yl)-methyl]amino]butyl}-1,2-benzoisothiazol-3-(2H)-on-1,1-dioxide or a pharmaceutically acceptable salt thereof dose-dependently reduced the severity of dystonic symptoms.

To demonstrate the efficacy of the compounds according to the invention in dystonic syndromes, a double-blind, placebo-controlled study is performed in patients with cervical dystonia (spasmodic torticollis) who do not tolerate injection of botulinum toxin. (-)-(R)-2-{4-[[3,4-dihydro-2H-benzopyran-2-yl)-methyl]amino]butyl}-1,2-benzoisothiazol-3-(2H)-on-1,1-dioxide monohydrochloride is titrated as described above in the range from 2.5 mg to 20 mg b.i.d. The Toronto western spasmodic torticollis rating scale (TWSTRS, C.L. Comella et al., 1997, Movement Disord, **12:** 570-575) is used as primary outcome measure. A significant improvement in the TWSTRS scores is noted for the patients treated with (-)-(R)-2-{4-[[3,4-dihydro-2H-benzopyran-2-yl)-methyl]amino]butyl}-1,2-benzoisothiazol-3-(2H)-on-1,1-dioxide or one of its pharmaceutically acceptable salts.

A preferred salt of (-)-(R)-2-{4-[[3,4-dihydro-2H-benzopyran-2-yl)-methyl]amino]butyl}-1,2-benzoisothiazol-3-(2H)-on-1,1 -dioxide is (-)-(R)-2-{4-[[3,4-dihydro-2H-benzopyran-2-yl)-methyl]amino]butyl}-1,2-benzoisothiazol-3-(2H)-on-1,1-dioxide monohydrochloride.

Therefore the invention relates to the use for the manufacture of a medicament for the treatment of dystonic syndromes, in particular of spasmalic torticollis and/or blepharospasm, in which the pharmacologically acceptable salt is (-)-(R)-2-{4-[[3,4-dihydro-2H-benzopyran-2-yl)-methyl]amino]butyl}-1,2-benzoisothiazol-3-(2H)-on-1,1-dioxide monohydrochloride.

Additionally, the invention relates to the use of a pharmaceutical composition containing at least one compound of (-)-(R)-2-{4-[[3,4-dihydro-2H-benzopyran-2-yl)-methyl]amino]butyl}-1,2-benzoisothiazol-3-(2H)-on-1,1-dioxide or one of its biocompatible salts for the treatment of dystonic syndromes.

The present invention relates to the use of substituted aminomethyl chromans of formula I 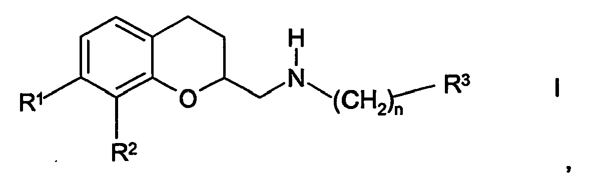 in which
R¹ represents hydrogen,
R² represents hydrogen, hydroxyl or a radical of the formula -OCH₃, -OCH₂CH₃, -OCH(CH₃)₂ or -CH₂C(CH₃)₂-Cl, or
R¹ and R² together form a radical of the formula 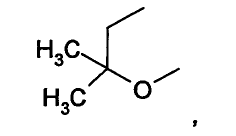
R³ represents cyclopentyl, cyclohexyl, cycloheptyl, or the following radical, designated as o-benzenesulphimidyl: 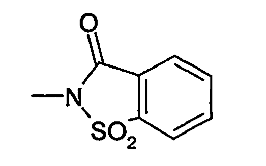
and
n is selected from 1, 2, 3, 4 or 5,
and their optical isomers and pharmaceutically acceptable salts for the manufacture of a medicament for the treatment of extrapyramidal symptoms induced by neuroleptics.

A particularly preferred compound of formula I is (-)-(R)-2-{4-[[3,4-dihydro-2H-benzopyran-2-yl)-methyl]amino]butyl}-1,2-benzoisothiazol-3-(2H)-on-1,1-dioxide or a physiologically acceptable salt thereof.

Therefore, the invention relates to the use of (-)-(R)-2-(4-[[3,4-dihydro-2H-benzopyran-2-yl)-methyl]amino]butyl}-1,2-benzoisothiazol-3-(2H)-on-1,1-dioxide or a pharmacologically acceptable salt for the manufacture of a medicament for the treatment of extrapyramidal symptoms induced by neuroleptics.

Extrapyramidal motoric disturbances induced by neuroleptics are e.g. early dyskinesia, dystonia, akathisia, parkinsonoid, in particular bradykinesia, or tardive dyskinesia.

(-)-(R)-2-{4-[[3,4-dihydro-2H-benzopyran-2-yl)-methyl]amino]butyl}-1,2-benzoisothiazol-3-(2H)-on-1,1-dioxide or a physiologically acceptable salt thereof are useful particularly for the treatment of akathisia and/or tardive dyskinesia and/or parkinsonoid.

A typical animal model is neuroleptics-induced muscle rigidity in rats according to S. Wolfarth *et al.,* Arch. Pharmacol. 1992; 345: 209-212. Rats are challenged with the conventional neuroleptic drug haloperidol which enhances muscle tone. Muscle tone is electromechanically measured as the resistence to passive flexion and extension of the hind limb. (-)-(R)-2-{4-[[3,4-dihydro-2H-benzopyran-2-yl)-methyl]amino]butyl}-1,2-benzoisothiazol-3-(2H)-on-1,1-dioxide or a pharmaceutically acceptable salt thereof decreased the muscle tone enhanced by haloperidol.

Another typical animal model is the neuroleptics sensitized monkey according to D.E. Casey, Psychopharmacology, 1996; **124**: 134-140. Monkeys treated repeatedly with conventional neuroleptics are highly sensitive to a subsequent challenge dose of neuroleptic drugs. When challenged, the monkeys immediately show extrapyramidal motor side effects such as dystonia, dyskinesias, akathisia, and bradykinesia which are rated by a scoring system. The conventional neuroleptic drug haloperidol is given as a challenge. When the before-mentioned extrapyramidal motor side effects occur, (-)-(R)-2-{4-[[3,4-dihydro-2H-benzopyran-2-yl)-methyl]amino]butyl}-1,2-benzoisothiazol-3-(2H)-on-1,1-dioxide or a pharmaceutically acceptable salt thereof is administered; (-)-(R)-2-{4-[[3,4-dihydro-2H-benzopyran-2-y))-methyl]amino]butyl}-1,2-benzoisothiazol-3-(2H)-on-1,1-dioxide dose-dependently reduce the extrapyramidal motor side effects.

Tardive dyskinesia is a common adverse effect of long-term treatment with neuroleptics. A typical study to investigate the efficacy of the compounds according to the invention in tardive dyskinesia is described in the following. 32 schizophrenic (DSM-III-R) inpatients aged 25 - 60 years on long-term stable antipsychotic treatment (duration of at least 5 years) entered the study. (-)-(R)-2-{4-[[3,4-dihydro-2H-benzopyran-2-yl)-methyl]amino]butyl}-1,2-benzoisothiazol-3-(2H)-on-1,1-dioxide monohydrochloride or placebo is administered as "add on" to the antipsychotic treatment, which is kept constant during the whole study. The dose of blinded medication is titrated over a period of 3 weeks in a range from 1 to 20 mg t.i.d. Then the medication is maintained under double-blind conditions for 2 weeks. After a 2-week wash-out period, the test drugs are crossed over. Assessments of tardive dyskinesia by means of the Abnormal Involuntary Movement Scale (AIMS, see obove) and of Parkinsonian extrapyramidal side effects (UPDRS, see above) are made pretreatment and posttreatment. AIMS scores during treatment with (-)-(R)-2-{4-[[3,4-dihydro-2H-benzopyran-2-yl)-methyl]amino]butyl}-1,2-benzoisothiazol-3-(2H)-on-1,1-dioxide monohydrochloride are significantly lower than during placebo period.

A preferred salt of (-)-(R)-2-{4-[[3,4-dihydro-2H-benzopyran-2-yl)-methyl]amino]butyl}-1,2-benzoisothiazol-3-(2H)-on-1,1-dioxide is (-)-(R)-2-{4-[[3,4-dihydro-2H-benzopyran-2-yl)-methyl]amino]butyl}-1,2-benzoisothiazol-3-(2H)-on-1,1-dioxide monohydrochloride.

Therefore the invention relates to the use for the manufacture of a medicament for the treatment of extrapyramidal symptoms induced by neuroleptics, in particular of akathisia and/or tardive dyskinesia, in which the pharmacologically acceptable salt is (-)-(R)-2-{4-[[3,4-dihydro-2H-benzopyran-2-yl)-methyl]amino]butyl}-1,2-benzoisothiazol-3-(2H)-on-1,1-dioxide monohydrochloride. '

Additionally, the invention relates to the use of a pharmaceutical composition containing at least one compound of (-)-(R)-2-{4-[[3,4-dihydro-2H benzopyran-2-yl)- methyl]amino]butyl)-1,2-benzoisothiazol-3-(2H)-on-1,1-dioxide or one of its biocompatible salts for the treatment of extrapyramidal symptoms induced by neuroleptics.

The present invention relates to the use of substituted aminomethyl chromans of formula I 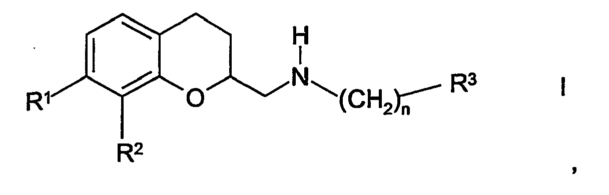 in which
R¹ represents hydrogen,
R² represents hydrogen, hydroxyl or a radical of the formula -OCH₃, -OCH₂CH₃, -OCH(CH₃)₂ or -CH₂C(CH₃)₂-Cl, or
R¹ and R² together form a radical of the formula 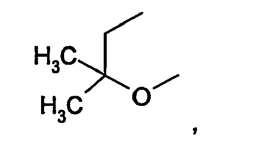
R³ represents cyclopentyl, cyclohexyl, cycloheptyl, or the following radical, designated as o-benzenesulphimidyl: 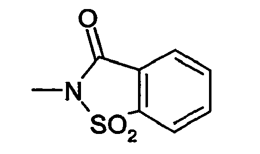
and
n is selected from 1, 2, 3, 4 or 5,
and their optical isomers and pharmaceutically acceptable salts for the manufacture of a medicament for the treatment of extrapyramidal movement disorders chosen from the group consisting of Gilles de la Tourette syndrome, ballism, myoclonus, restless legs syndrome and Wilson's disease.

A particularly preferred compound of formula I is (-)-(R)-2-{4-[[3,4-dihydro-2H-benzopyran-2-yl)-methyl]amino]butyl}-1,2-benzoisothiazol-3-(2H)-on-1,1-dioxide or a physiologically acceptable salt thereof.

Therefore, the invention relates to the use of (-)-(R)-2-{4-[[3,4-dihydro-2H-benzopyran-2-yl)-methyl]amino]butyl}-1,2-benzoisothiazol-3-(2H)-on-1,1-dioxide or a pharmacologically acceptable salt for the manufacture of a medicament for the treatment of extrapyramidal movement disorders chosen from the group consisting of Gilles de la Tourette syndrome, ballism, myoclonus, restless legs syndrome and Wilson's disease.

A typical animal model for myoclonus is myoclonus induced by an acute hypoxic episode according to D.D. Truong *et al.,* Mov. Dsiord., 1994; **9:** 201-206). In this model of posthypoxic myoclonus, rats undergo a cardiac arrest for 8 minutes and are resuscitated thereafter. Myoclonic jerks occur spontaneously but can be provoked by auditory stimulation, too, worsening over the days following cardiac arrest. (-)-(R)-2-{4-[[3,4-dihydro-2H-benzopyran-2-yl)-methyl]amino]butyl}-1,2-benzoisothiazol-3-(2H)-on-1,1 - dioxide or one of its pharmacologically acceptable salts dose-dependently reduce the number of spontaneous and autitory-evoked myoclonic jerks,

A preferred salt of (-)-(R)-2-{4-[[3,4-dihydro-2H-benzopyran-2-yl)-methyl]amino]butyl}-1,2-benzoisothiazol-3-(2H)-on-1,1-dioxide is (-)-(R)-2-{4-[[3,4-dihydro-2H-benzopyran-2-yl)-methyl]amino]butyl}-1,2-benzoisothiazol-3-(2H)-on-1,1-dioxide monohydrochloride.

Therefore the invention relates to the use for the manufacture of a medicament for the treatment of extrapyramidal movement disorders chosen from the group consisting of Gilles de la Tourette syndrome, ballism, myoclonus, restless legs syndrome and Wilson's disease in which the pharmacologically acceptable salt is (-)-(R)-2-{4-[[3,4-dihydro-2H-benzopyran-2-yl)-methyl]amino]butyl}-1,2-benzoisothiazol-3-(2H)-on-1,1-dioxide monohydrochloride.

Additionally, the invention relates to the use of a pharmaceutical composition containing at least one compound of (-)-(R)-2-{4-[[3,4-dihydro-2H-benzopyran-2-yl)-methyl]amino]butyl}-1,2-benzoisothiazol-3-(2H)-on-1,1-dioxide or one of its biocompatible salts for the treatment of extrapyramidal movement disorders chosen from the group consisting of Gilles de la Tourette syndrome, ballism, myoclonus, restless legs syndrome and Wilson's disease.

The extrapyramidal movement disorders such as Steele-Richardson-Olszewski syndrome (= progressive supranuclear palsy), cortico-basal degeneration, olivo-ponto cerebellar atrophy, Shy Drager syndrome, minor chorea, chorea of pregnancy, writer's cramp, blepharospasm, Meige syndrome, dopa-sensitive dystonia, Gilles de la Tourette syndrome, ballism, myoclonus, restless legs syndrome, and Wilson's disease are not frequent enough to perform regular double-blind trials. However, the medical need in this field is pressing since no sufficient therapies are available so far.
Therefore, open-label observations in few selected patients are an adequate method to demonstrate the efficacy of (-)-(R)-2-{4-[[3,4-dihydro-2H-benzopyran-2-yl)-methyl]amino]butyl}-1,2-benzoisothiazol-3-(2H)-on-1,1-dioxide or a physiologically acceptable salt thereof.

All the pharmaceutical preparations used for the treatment of extrapyramidal movement disorders and/or for the treatment of adverse effects of anti-Parkinsonian drugs in extrapyramidal movement disorders including the medicinal combination can be used as pharmaceuticals in human or veterinary medicine.

The compositions of the invention are preferably administered parenterally, or better still orally, although the other routes of administration, for instance such as rectal administration, are not excluded.

Suitable excipients are organic or inorganic substances which are suitable for enteral (e.g. oral), parenteral or topical adminstration and which do not react with (-)-(R)-2-{4-[[3,4-dihydro-2H-benzopyran-2-yl)-methyl]amino]butyl}-1,2-benzoisothiazol-3-(2H)-on-1,1-dioxide and/or one of its biocompatible salts, for example water, vegetable oils, benzyl alcohols, alkylene glycols, polyethylene glycols, glycerol triacetate, gelatine, carbohydrates such as lactose or starch, magnesium stearate, talc, petroleum jelly. Forms which are used for oral administration are, in particular, tablets, pills, sugar-coated tablets, capsules, powders, granules, syrups, liquids or drops, forms for rectal administration are, in particular suppositories, forms for parenteral administration are, in particular, solvents, preferably oily or aqueous solutions, furthermore suspensions, emulsions or implants, and forms for topical administration are transdermal plasters, ointments, creams or powders. (-)-(R)-2-{4-[[3,4-dihydro-2H-benzopyran-2-yl)-methyl]amino]butyl}-1,2-benzoisothiazol-3-(2H)-on-1,1 - dioxide and/or one of its pharmaceutically acceptable salts may also be lyophilized and the resulting lyophilisates used for example for the preparation of injectable products. The abovementioned preparations can be in sterilized form and/or comprise auxiliaries such as glidants, preservatives, stabilizers and/or wetting agents, emulsifiers, salts for modifying the osmotic pressure, buffer substances, colourings, flavourings and/or other active ingredients, e.g. one or more vitamins. Preparations may, if desired, be designed to give slow release of (-)-(R)-2-{4-[[3,4-dihydro-2H-benzopyran-2-yl)-methyl]amino]butyl}-1,2-benzoisothiazol-3-(2H)-on-1,1-dioxide or a biocompatible salt thereof.

The examples which follow relate to pharmaceutical products:

### Example A: Vials

A solution of 100 g of (-)-(R)-2-{4-[[3,4-dihydro-2H-benzopyran-2-yl)-methyl]amino]butyl}-1,2-benzoisothiazol-3-(2H)-on-1,1-dioxide or a physiologically acceptable salt thereof and 5 g of disodium hydrogen phosphate in 3 I of twice-distilled water is brought to pH 6.5 with 2N hydrochloric acid, filter-sterilized, filled into vials, lyophilized under sterile conditions and sealed in sterile form. Each vial comprises 5 mg of active ingredient.

### Example B: Suppositories

A mixture of 20 g of (-)-(R)-2-{4-[[3,4-dihydro-2H-benzopyran-2-yl)-methyl]amino]butyl}-1,2-benzoisothiazol-3-(2H)-on-1,1-dioxide or a physiologically acceptable salt thereof is melted with 100 g of soya lecithin and 1400 g of cocoa butter, and the mixture is poured into moulds and left to cool. Each suppository comprises 20 mg of active ingredient.

### Example C: Solution

A solution is prepared from 1 g of (-)-(R)-2-{4-[[3,4-dihydro-2H-benzopyran-2-ylrmethyl]amino]butyl}-1,2-benzoisothiazol-3-(2H)-on-1,1-dioxide or a physiologically acceptable salt thereof, 9.38 g of NaH₂PO₄-2H₂0, 28.48 g of Na₂HPO₄·12H₂O and 0.1 g of benzalkonium chloride in 940 ml of twice-distilled water. The pH is brought to 6.8, and the solution is made up to 1 I and sterilized by irradiation. This solution can be used in the form of eyedrops.

### Example D: Ointment

500 mg of (-)-(R)-2-{4-[[3,4-dihydro-2H-benzopyran-2-yl)-methyl]amino]butyl}-1,2-benzoisothiazol-3-(2H)-on-1,1-dioxide or a physiologically acceptable salt thereof are mixed with 99.5 g of petroleum jelly under aseptic conditions.

### Example E-1: Tablets

A mixture of 1 kg of (-)-(R)-2-{4-[[3,4-dihydro-2H-benzopyran-2-yl)-methyl]amino]butyl}-1,2-benzoisothiazol-3-(2H)-on-1,1-dioxide or a physiologically acceptable salt thereof, 4 kg of lactose, 1.2 kg of potato starch, 0.2 kg of talc and 0.1 kg of magnesium stearate is tableted in the customary manner in such a way that each tablet comprises 10 mg of active ingredient.

### Example E-2: Tablets

A mixture of 100 g of (-)-(R)-2-{4-[[3,4-dihydro-2H-benzopyran-2-yl)-methyl]amino]butyl)-1,2-benzoisothiazol-3-(2H)-on-1,1-dioxide monohydrochloride, 1 kg of I-dopa, 250 g benserazide, 4 kg of lactose, 1.6 kg of potato starch, 0.2 kg of talc and 0.1 kg of magnesium stearate is tableted in the customary manner in such a way that each tablet comprises 5 mg (-)-(R)-2-{4-[[3,4-dihydro-2H-benzopyran-2-yl)-methyl]amino]butyl}-1,2-benzoisothiazol-3-(2H)-on-1,1-dioxide monohydrochloride, 50 mg of I-dopa and 12,5 mg benserazide.

### Example F: Sugar-coated tablets

A mixture is tableted analogously to Example E, and the tablets are subsequently coated in the customary manner with a coating of sucrose, potato starch, talc, tragacanth and colouring.

### Example G: capsules

2 kg of (-)-(R)-2-{4-[[3,4-dihydro-2H-benzopyran-2-yl)-methyl]amino]butyl}-1,2-benzoisothiazol-3-(2H)-on-1,1-dioxide or a physiologically acceptable salt thereof are filled into hard gelatin capsules in the customary manner so that each capsule comprises 20 mg of the active ingredient.

### Example H: Ampoules

A solution of 1 kg of (-)-(R)-2-{4-[[3,4-dihydro-2H-benzopyran-2-yl)-methyl]amino]butyl}-1,2-benzoisothiazol-3-(2H)-on-1,1-dioxide or a physiologically acceptable salt thereof in 60 I of twice-distilled water is filter-sterilized, filled into ampoules, lyophilized under sterile conditions and sealed in sterile form. Each ampoule comprises 10 mg of active ingredient.

### Example I: Spray for inhalation

14 g of (-)-(R)-2-{4-[[3,4-dihydro-2H-benzopyran-2-yl)-methyl]amino]butyl}-1,2-benzoisothiazol-3-(2H)-on-1,1-dioxide or a physiologically acceptable salt thereof are dissolved in 10 I of isotonic NaCl solution, and the solution is filled into commercially available pump-operated spray containers. The solution can be sprayed into mouth or nose. One actuation (approximately 0.1 ml) corresponds to a dose of approximately 0.14 mg.

## Claims

1. Use of substituted aminomethyl chromans of formula I 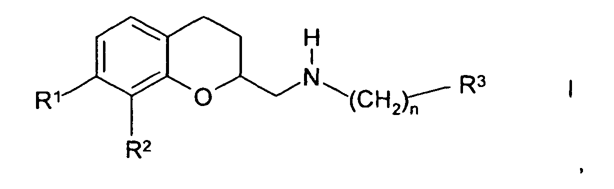 in which
R¹ represents hydrogen,
R² represents hydrogen, hydroxyl or a radical of the formula -OCH₃, -OCH₂CH₃, -OCH(CH₃)₂ or -CH₂C(CH₃)₂-Cl, or
R¹ and R² together form a radical of the formula 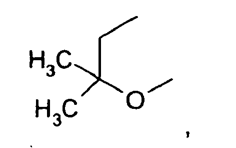
R³ represents cyclopentyl, cyclohexyl, cycloheptyl, or the following radical, designated as o-benzenesulphimidyl: 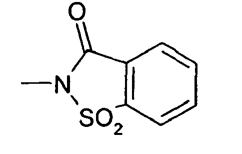
and
n is selected from 1, 2, 3, 4 or 5,
and their optical isomers and pharmaceutically acceptable salts for the manufacture of a medicament for the treatment of adverse effects of anti-Parkinsonian drugs in idiopathic Parkinson's disease.

2. Use according to claim 1, in which the compound of formula I is (-)-(R)-2-{4-[[3,4-dihydro-2H-benzopyran-2-yl)-methyl]amino]butyl}-1,2-benzoisothiazol-3-(2H)-on-1,1-dioxide or a physiologically acceptable salt thereof.

3. Use according to claim 1 or 2 **characterized in that** the adverse effects of anti-Parkinsonian drugs in idiopathic Parkinson's disease are motor fluctuations.

4. Use according to claim 1 or 2 **characterized in that** the adverse effects of anti-Parkinsonian drugs in idiopathic Parkinson's disease are dyskinesias.

5. Use according to claim 4 **characterized in that** the said dyskinesia is treatment-associated dyskinesia in Parkinson's disease.

6. Use according to claim 4 **characterized in that** the said dyskinesia is levodopa-induced dyskinesia in Parkinson's disease.

7. Use according to claim 1 - 6 **characterized in that** the said anti-Parkinsonian drug is chosen from a group consisting of L-Dopa (levodopa), L-Dopa combined with benserazide or carbidopa, dopamine agonists such as bromocriptine apomorphine, cabergoline, pramipexol, ropinirol, pergolide, dihydro-α-ergocriptine or lisuride plus all drugs acting via stimulation of dopamine receptors, inhibitors of catechol-O-methyl transferase (COMT) such as entacapone or tolcapone, inhibitors of monoamine oxidase (MAO) such as selegiline and antagonists of N-methyl-D-aspartate (NMDA) receptors such as amantadine or budipine.

8. Use according to claim 1 - 7 **characterized in that** the said anti-Parkinsonian drug is L-Dopa.

9. Use of substituted aminomethyl chromans of formula I 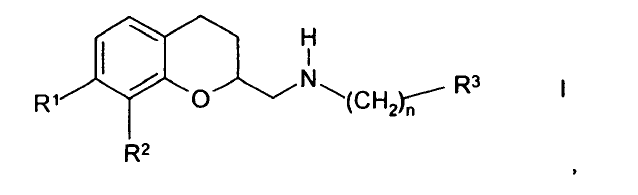 in which
R¹ represents hydrogen,
R² represents hydrogen, hydroxyl or a radical of the formula -OCH₃, -OCH₂CH₃, -OCH(CH₃)₂ or -CH₂C(CH₃)₂-Cl, or
R¹ and R² together form a radical of the formula 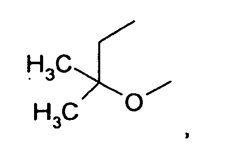
R³ represents cyclopentyl, cyclohexyl, cycloheptyl, or the following radical, designated as o-benzenesulphimidyl: 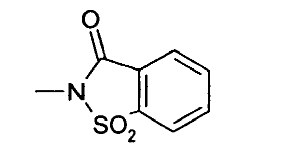
and
n is selected from 1, 2, 3, 4 or 5,
and their optical isomers and pharmaceutically acceptable salts for the manufacture of a medicament for the treatment of adverse effects of anti-Parkinsonian drugs in Parkinson's syndromes.

10. Use according to claim 9, in which the compound of formula I is (-)-(R)-2-{4-[[3,4-dihydro-2H-benzopyran-2-yl)-methyl]amino]butyl}-1,2-benzoisothiazol-3-(2H)-on-1,1-dioxide or a physiologically acceptable salt thereof.

11. Use according to claim 9 or 10 **characterized in that** the said anti-Parkinsonian drug is chosen from a group consisting of L-Dopa (levodopa), L-Dopa combined with benserazide or carbidopa, dopamine agonists such as bromocriptine, apomorphine, cabergoline, pramipexol, ropinirol, pergolide, dihydro-α-ergocriptine or lisuride plus all drugs acting via stimulation of dopamine receptors, inhibitors of catechol-O-methyl transferase (COMT) such as entacapone or tolcapone, inhibitors of monoamine oxidase (MAO) such as selegiline and antagonists of N-methyl-D-aspartate (NMDA) receptors such as amantadine or budipine.

12. Use according to claim 9 - 11 **characterized in that** the said anti-Parkinsonian drug is L-Dopa.

13. Use of substituted aminomethyl chromans of formula I 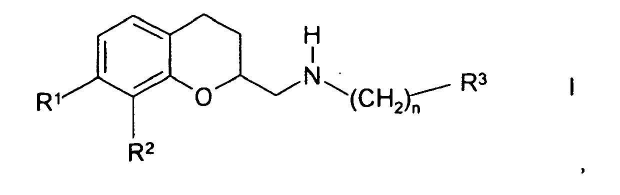 in which
R¹ represents hydrogen,
R² represents hydrogen, hydroxyl or a radical of the formula -OCH₃, -OCH₂CH₃, -OCH(CH₃)₂ or -CH₂C(CH₃)₂-Cl, or
R¹ and R² together form a radical of the formula 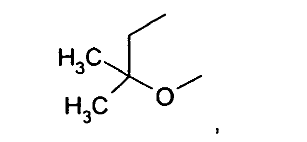
R³ represents cyclopentyl, cyclohexyl, cycloheptyl, or the following radical, designated as o-benzenesulphimidyl: 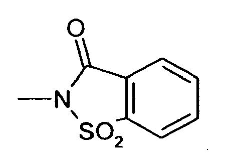
and
n is selected from 1, 2, 3, 4 or 5,
and their optical isomers and pharmaceutically acceptable salts for the manufacture of a medicament for the treatment of extrapyramidal symptoms induced by neuroleptics.

14. Use according to claim 13, in which the compound of formula I is (-)-(R)-2-{4-[[3,4-dihydro-2H-benzopyran-2-yl)-methyl]amino]butyl}-1,2-benzoisothiazol-3-(2H)-on-1,1-dioxide or a physiologically acceptable salt thereof.

15. Use according to claims 13 or 14, in which the extrapyramidal symptom induced by neuroleptics is tardive dyskinesia.

16. Pharmaceutical composition comprising, as active principles,
(i) a compound of formula I 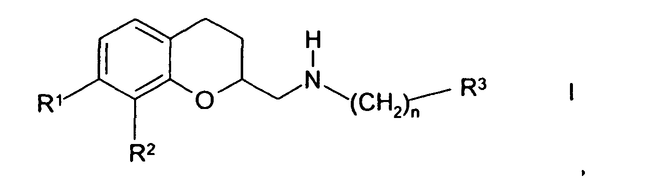 in which
R¹ represents hydrogen,
R² represents hydrogen, hydroxyl or a radical of the formula -0CH₃, -OCH₂CH₃, -OCH(CH₃)₂ or -CH₂C(CH₃)₂-Cl, or
R¹ and R² together form a radical of the formula 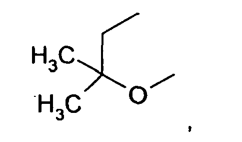
R³ represents cyclopentyl, cyclohexyl, cycloheptyl, or the following radical, designated as o-benzenesulphimidyl: 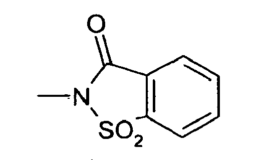
and
n is selected from 1, 2, 3, 4 or 5,
or one of their optical isomers or pharmaceutically acceptable salts,
and (ii) at least one anti-Parkinsonian drug, in combination with one or more pharmaceutically acceptable excipients.

17. Composition according to claim 16, in which (i) the active principle is (-)-(R)-2-{4-[[3,4-dihydro-2H-benzopyran-2-yl)-methyl]amino]butyl}-1,2-benzoisothiazol-3-(2H)-on-1,1-dioxide or a physiologically acceptable salt thereof, and (ii) at least one anti-Parkinsonian drug, in combination with one or more pharmaceutically acceptable excipients.

18. Composition according to claims 16 or 17, in which the at least one conventional anti-Parkinsonian drug is chosen from a group consisting of L-Dopa (levodopa), L-Dopa combined with benserazide or carbidopa, dopamine agonists such as bromocriptine, apomorphine, cabergoline, pramipexol, ropinirol, pergolide, dihydro-α-ergocriptine or lisuride plus all drugs acting via stimulation of dopamine receptors, inhibitors of catechol-O-methyl transferase (COMT) such as entacapone or tolcapone, inhibitors of monoamine oxidase (MAO) such as selegiline and antagonists of N-methyl-D-aspartate (NMDA) receptors such as amantadine or budipine.

19. Composition according to claim 16 - 18, in which (i) the active principle is in the form of its monohydrochloride and (ii) the conventional anti-Parkinsonian drug is L-Dopa.

## Patentansprüche

1. Verwendung von substituierten Aminomethylchromanen der Formel I 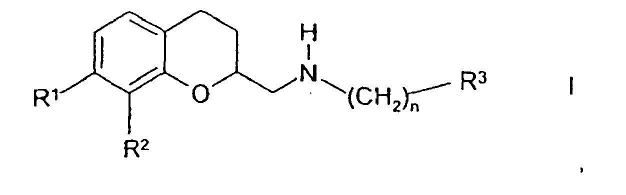 wobei
R¹ Wasserstoff bedeutet,
R² Wasserstoff, Hydroxyl oder einen Rest der Formel -OCH₃, -OCH₂CH₃, -OCH(CH₃)₂ oder -CH₂C(CH₃)₂-Cl bedeutet, oder
R¹ und R² zusammen einen Rest der Formel 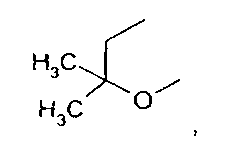 bilden,
R³ Cyclopentyl, Cyclohexyl, Cycloheptyl oder den folgenden Rest bedeutet, der als o-Benzolsulphimidyl bezeichnet wird: 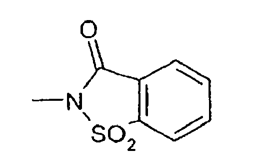
und
n aus 1, 2, 3, 4 oder 5 ausgewählt ist,
und ihrer optischen Isomere und pharmazeutisch unbedenklichen Salze zur Herstellung eines Arzneimittels zur Behandlung von Nebenwirkungen von Anti-Parkinson-Medikamenten bei der idiopathischen Parkinson-Krankheit.

2. Verwendung nach Anspruch 1, wobei die Verbindung der Formel I (-)-(R)-2-{4-[[3,4-Dihydro-2H-benzopyran-2-yl)-methyl]amino]butyl}-1,2-benzoisothiazol-3-(2H)-on-1,1-dioxid oder ein physiologisch unbedenkliches Salz davon ist.

3. Verwendung nach Anspruch 1 oder 2, **dadurch gekennzeichnet, dass** die Nebenwirkungen der Anti-Parkinson-Medikamente bei der idiopathischen Parkinson-Krankheit motorische Fluktuationen sind.

4. Verwendung nach Anspruch 1 oder 2, **dadurch gekennzeichnet, dass** die Nebenwirkungen der Anti-Parkinson-Medikamente bei der idiopathischen Parkinson-Krankheit Dyskinesien sind.

5. Verwendung nach Anspruch 4, **dadurch gekennzeichnet, dass** die Dyskinesie behandlungsassoziierte Dyskinesie bei der Parkinson-Krankheit ist.

6. Verwendung nach Anspruch 4, **dadurch gekennzeichnet, dass** die Dyskinesie lävodopa-induzierte Dyskinesie bei der Parkinson-Krankheit ist.

7. Verwendung nach Anspruch 1 bis 6, **dadurch gekennzeichnet, dass** das Anti-Parkinson-Medikament aus einer Gruppe, bestehend aus L-Dopa (Lävodopa), L-Dopa in Kombination mit Benserazid oder Carbidopa, Dopamin-Agonisten, wie Bromcriptin, Apomorphin, Cabergolin, Pramipexol, Ropinirol, Pergolid, Dihydro-α-ergocriptin oder Lisurid zuzüglich sämtlicher Medikamente, die über eine Stimulation der Dopaminrezeptoren, Inhibitoren der Catechol-O-methyltransferase (COMT), wie Entacapon oder Tolcapon, Inhibitoren der Monoaminoxidase (MAO), wie Selegilin, und Antagonisten von N-Methyl-D-aspartat (NMDA)-Rezeptoren, wie Amantadin oder Budipin, wirken, ausgewählt ist

8. Verwendung nach Anspruch 1 bis 7, **dadurch gekennzeichnet, dass** das Anti-Parkinson-Medikament L-Dopa ist.

9. Verwendung substituierter Aminomethylchromane der Formel I 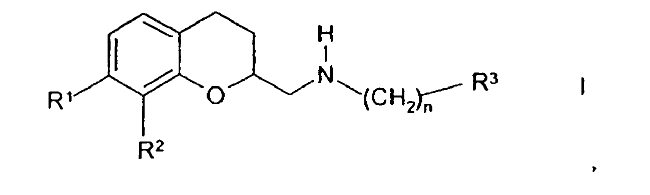 wobei
R¹ Wasserstoff bedeutet,
R²Wasserstoff, Hydroxyl oder einen Rest der Formel -OCH₃, -OCH₂CH₃, -OCH(CH₃)₂ oder -CH₂C(CH₃)₂-Cl bedeutet, oder
R¹ und R² zusammen einen Rest der Formel 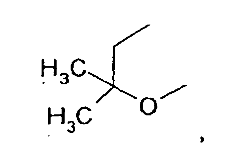 bilden,
R³ Cyclopentyl, Cyclohexyl, Cycloheptyl oder den folgenden Rest bedeutet, der als o-Benzolsulphimidyl bezeichnet wird: 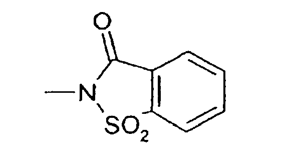
und
n aus 1, 2, 3, 4 oder 5 ausgewählt ist,
und ihrer optischen Isomere und pharmazeutisch unbedenklichen Salze zur Herstellung eines Arzneimittels zur Behandlung von Nebenwirkungen von Anti-Parkinson-Medikamenten bei Parkinson-Syndromen.

10. Verwendung nach Anspruch 9, wobei die Verbindung der Formel I (-)-(R)-2-{4-[[3,4-Dihydro-2H-benzopyran-2-yl)-methyl]amino]butyl}-1,2-benzoisothiazol-3-(2H)-on-1,1-dioxid oder ein physiologisch unbedenkliches Salz davon ist.

11. Verwendung nach Anspruch 9 oder 10, **dadurch gekennzeichnet, dass** das Anti-Parkinson-Medikament aus der Gruppe, bestehend aus L-Dopa (Lävodopa), L-Dopa in Kombination mit Benserazid oder Carbidopa, Dopamin-Agonisten, wie Bromcriptin, Apomorphin, Cabergolin, Pramipexol, Ropinirol, Pergolid, Dihydro-α-ergocriptin oder Lisurid zuzüglich sämtlicher Medikamente, die über eine Stimulation der Dopaminrezeptoren, Inhibitoren der Catechol-O-methyltransferase (COMT), wie Entacapon oder Tolcapon, Inhibitoren der Monoaminoxidase (MAO), wie Selegilin, und Antagonisten von N-Methyl-D-aspartat (NMDA)-Rezeptoren, wie Amantadin oder Budipin, wirken, ausgewählt ist.

12. Verwendung nach Anspruch 9 bis 11, **dadurch gekennzeichnet, dass** das Anti-Parkinson-Medikament L-Dopa ist.

13. Verwendung substituierter Aminomethylchromane der Formel I 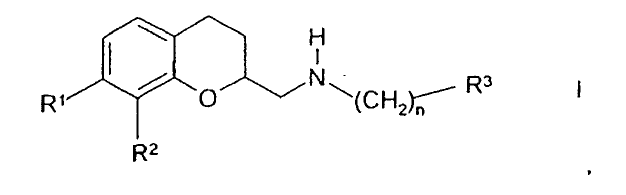 wobei
R¹ Wasserstoff bedeutet,
R² Wasserstoff, Hydroxyl oder einen Rest der Formel -OCH₃, -OCH₂CH₃, -OCH(CH₃)₂ oder -CH₂C(CH₃)₂-Cl bedeutet, oder
R¹ und R² zusammen einen Rest der Formel 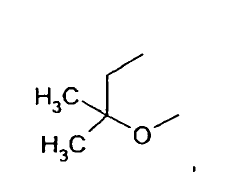 bilden,
R³ Cyclopentyl, Cyclohexyl, Cycloheptyl oder den folgenden Rest bedeutet, der als o-Benzolsulphimidyl bezeichnet wird: 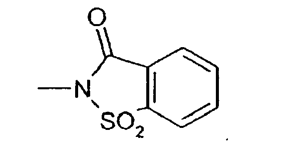
und
n aus 1, 2, 3, 4 oder 5 ausgewählt ist,
und ihrer optischen Isomere und pharmazeutisch unbedenklichen Salze zur Herstellung eines Arzneimittels zur Behandlung von extrapyramidalen Symptomen, die durch Neuroleptika induziert werden.

14. Verwendung nach Anspruch 13, wobei die Verbindung der Formel I (-)-(R)-2-{4-[[3,4-Dihydro-2H-benzopyran-2-yl)-methyl]amino]butyl}-1,2-benzoisothiazol-3-(2H)-on-1,1-dioxid oder ein physiologisch unbedenkliches Salz davon ist.

15. Verwendung nach Anspruch 13 oder **14,** wobei das durch Neuroleptika induzierte extrapyramidale Symptom tardive Dyskinesie ist.

16. Pharmazeutische Zusammensetzung, enthaltend als Wirkstoff
(i) eine Verbindung der Formel I 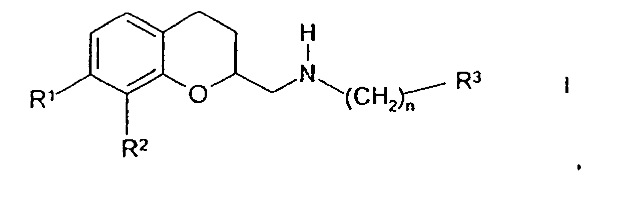 wobei
R¹ Wasserstoff bedeutet,
R² Wasserstoff, Hydroxyl oder einen Rest der Formel -OCH₃, -OCH₂CH₃, -OCH(CH₃)₂ oder -CH₂C(CH₃)₂-Cl bedeutet, oder
R¹ und R² zusammen einen Rest der Formel 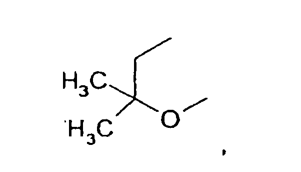 bilden,
R³ Cyclopentyl, Cyclohexyl, Cycloheptyl oder den folgenden Rest bedeutet, der als o-Benzolsulphimidyl bezeichnet wird: 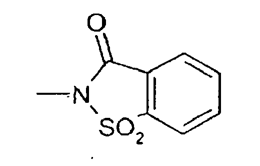
und
n aus 1, 2, 3, 4 oder 5 ausgewählt ist,
oder eines ihrer optischen Isomere oder pharmazeutisch unbedenklichen Salze, und
(ii) mindestens ein Anti-Parkinson-Medikament, in Kombination mit einem oder mehreren pharmazeutisch unbedenklichen Trägern.

17. Zusammensetzung nach Anspruch 16, wobei das Wirkstoff ist:
(i) (-)-(R)-2-(4-[[3,4-Dihydro-2H-benzopyran-2-yl)-methyl]amino]butyl}-1,2-benzoisothiazol-3-(2H)-on-1,1-dioxid oder ein physiologisch unbedenkliches Salz davon, und
(ii) mindestens ein Anti-Parkinson-Medikament, in Kombination mit einem oder mehreren pharmazeutisch unbedenklichen Trägern.

18. Zusammensetzung nach den Ansprüchen 16 oder 17, wobei mindestens ein übliches Anti-Parkinson-Medikament aus der Gruppe, bestehend aus L-Dopa (Lävodopa), L-Dopa in Kombination mit Benserazid oder Carbidopa, Dopamin-Agonisten, wie Bromcriptin, Apomorphin, Cabergolin, Pramipexol, Ropinirol, Pergolid, Dihydro-α-ergocriptin oder Lisurid zuzüglich sämtlicher Medikamente, die über eine Stimulation der Dopaminrezeptoren, Inhibitoren der Catechol-O-methyltransferase (COMT), wie Entacapon oder Tolcapon, Inhibitoren der Monoaminoxidase (MAO), wie Selegilin und Antagonisten von N-Methyl-D-aspartat (NMDA)-Rezeptoren, wie Amantadin oder Budipin, wirken, ausgewählt ist.

19. Verwendung nach Anspruch 16 bis 18, wobei (i) das Wirkstoff in der Form seines Monohydrochlorids vorliegt, und (ii) das übliche Anti-Parkinson-Medikament L-Dopa ist.

## Revendications

1. Utilisation d'aminométhylchromanes substituées de la formule I 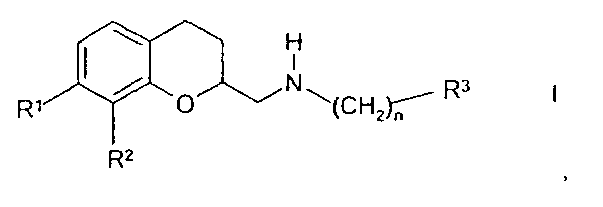 dans laquelle
R¹ représente hydrogène,
R² représente hydrogène, hydroxyle ou un radical de la formule -OCH₃, -OCH₂CH₃, -OCH(CH₃)₂ ou -CH₂C(CH₃)₂-Cl, ou
R¹ et R² forment ensemble un radical de la formule 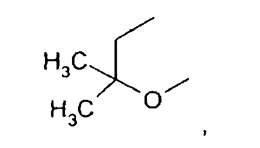
R³ représente cyclopentyle, cyclohexyle, cycloheptyle ou le radical qui suit, désigné en tant que o-benzènesulphimidyle : 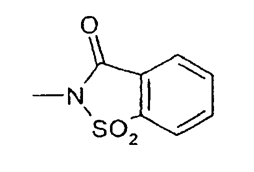
et
n est choisi parmi 1, 2, 3, 4 ou 5,
et de leurs isomères optiques et sels pharmaceutiquement acceptables pour la fabrication d'un médicament pour le traitement des effets défavorables de médicaments anti-Parkinsoniens au niveau de la maladie de Parkinson idiopathique.

2. Utilisation selon la revendication 1, dans laquelle le composé de la formule I est (-)-(R)-2-{4-[[3,4-dihydro-2H-benzopyran-2-yl)méthyl]-amino]butyl}-1,2-benzoisothiazol-3-(2H)-on-1,1-dioxyde ou son sel physiologiquement acceptable.

3. Utilisation selon la revendication 1 ou 2, **caractérisée en ce que** les effets défavorables des médicaments anti-Parkinsoniens au niveau de la maladie de Parkinson idiopathique sont des fluctuations motrices.

4. Utilisation selon la revendication 1 ou 2, **caractérisée en ce que** les effets défavorables des médicaments anti-Parkinsoniens au niveau de la maladie de Parkinson idiopathique sont des dyskinésies.

5. Utilisation selon la revendication 4, **caractérisée en ce que** ladite dyskinésie est une dyskinésie associée par traitement au niveau de la maladie de Parkinson.

6. Utilisation selon la revendication 4, **caractérisée en ce que** ladite dyskinésie est une dyskinésie induite par levodopa au niveau de la maladie de Parkinson.

7. Utilisation selon les revendications 1 à 6, **caractérisée en ce que** ledit médicament anti-Parkinsonien est choisi parmi un groupe comprenant L-Dopa (levodopa), L-Dopa combiné avec bensérazide ou carbidopa, des agonistes de dopamine tels que bromocriptine, apomorphine, cabergoline, pramipexol, ropinirol, pergolide, dihydro-α-ergocriptine ou lisuride plus tous les médicaments agissant via une stimulation de récepteurs de dopamine, des inhibiteurs de catéchol-O-méthyle transférase (COMT) tels que entacapone ou tolcapone, des inhibiteurs de monoamine oxydase (MAO) tels que sélégiline et des antagonistes de récepteurs de N-méthyl-D-aspartate (NMDA) tels que amantadine ou budipine.

8. Utilisation selon les revendications 1 à 7, **caractérisée en ce que** lesdits médicaments anti-Parkinsoniens sont L-Dopa.

9. Utilisation d'aminométhylchromanes substituées de la formule I 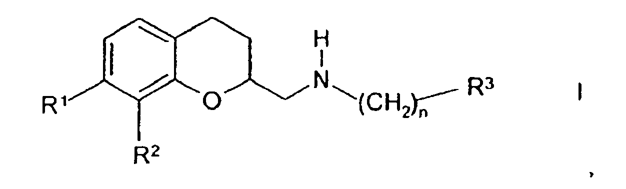 dans laquelle
R¹ représente hydrogène,
R² représente hydrogène, hydroxyle ou un radical de la formule -OCH₃, -OCH₂CH₃, -OCH(CH₃)₂ ou -CH₂C(CH₃)₂-Cl, ou
R¹ et R² forment ensemble un radical de la formule 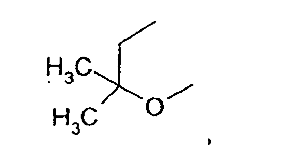
R³ représente cyclopentyle, cyclohexyle, cycloheptyle ou le radical qui suit, désigné en tant que o-benzènesulphimidyle : 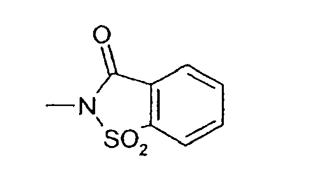
et
n est choisi parmi 1, 2, 3, 4 ou 5,
et de leurs isomères optiques et sels pharmaceutiquement acceptables pour la fabrication d'un médicament pour le traitement des effets défavorables de médicaments anti-Parkinsoniens au niveau de syndromes de Parkinson.

10. Utilisation selon la revendication 9, dans laquelle le composé de la formule 1 est (-)-(R)-2-{4-[[3,4-dihydro-2H-benzopyran-2-yl)méthyl]-amino]butyl}-1,2-benzoisothiazol-3-(2H)-on-1,1-dioxyde ou son sel physiologiquement acceptable.

11. Utilisation selon la revendication 9 ou 10, **caractérisée en ce que** ledit médicament anti-Parkinsonien est choisi parmi un groupe comprenant L-Dopa (levodopa), L-Dopa combiné avec bensérazide ou carbidopa, des agonistes de dopamine tels que bromocriptine, apomorphine, cabergoline, pramipexol, ropinirol, pergolide, dihydro-α-ergocriptine ou lisuride plus tous les médicaments agissant via une stimulation de récepteurs de dopamine, des inhibiteurs de catéchol-O-méthyle transférase (COMT) tels que entacapone ou tolcapone, des inhibiteurs de monoamine oxydase (MAO) tels que sélégiline et des antagonistes de récepteurs de N-méthyl-D-aspartate (NMDA) tels que amantadine ou budipine.

12. Utilisation selon les revendications 9 à 11, **caractérisée en ce que** ledit médicament anti-Parkinsonien est L-Dopa.

13. Utilisation d'aminométhylchromanes substituées de la formule I 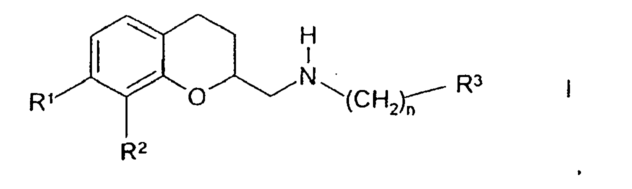 dans laquelle
R¹ représente hydrogène,
R² représente hydrogène, hydroxyle ou un radical de la formule -OCH₃, -OCH₂CH₃, -OCH(CH₃)₂ ou -CH₂C(CH₃)₂-Cl, ou
R¹ et R² forment ensemble un radical de la formule 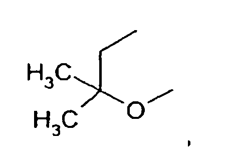
R³ représente cyclopentyle, cyclohexyle, cycloheptyle ou le radical qui suit, désigné en tant que o-benzènesulphimidyle : 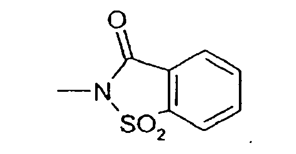
et
n est choisi parmi 1, 2, 3, 4 ou 5,
et de leurs isomères optiques et sels pharmaceutiquement acceptables pour la fabrication d'un médicament pour le traitement des symptômes extrapyramidaux induits par des neuroleptiques.

14. Utilisation selon la revendication 13, dans laquelle le composé de la formule I est (-)-(R)-2-{4-[[3,4-dihydro-2H-benzopyran-2-yl)méthyl]-amino]butyl}-1,2-benzoisothiazol-3-(2H)-one-1,1-dioxyde ou son sel physiologiquement acceptable.

15. Utilisation selon la revendication 13 ou 14, dans laquelle le symptôme extrapyramidal induit par des neuroleptiques est la diskynésie tardive.

16. Composition pharmaceutique comprenant, en tant que principes actifs,
(i) un composé de la formule I 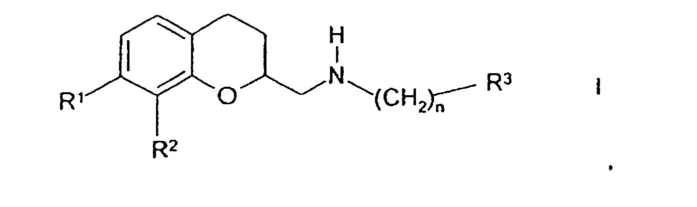 dans laquelle
R¹ représente hydrogène,
R² représente hydrogène, hydroxyle ou un radical de la formule -OCH₃, -OCH₂CH₃, -OCH(CH₃)₂ ou -CH₂C(CH₃)₂-Cl, ou
R¹ et R² forment ensemble un radical de la formule 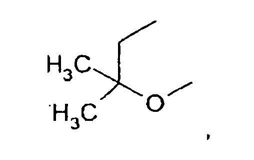
R³ représente cyclopentyle, cyclohexyle, cycloheptyle ou le radical qui suit, désigné en tant que o-benzènesulphimidyle : 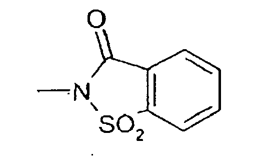
et
n est choisi parmi 1, 2, 3, 4 ou 5,
ou l'un de ses isomères optiques ou sels pharmaceutiquement acceptables
et (ii) au moins un médicament anti-Parkinsonien en combinaison avec un ou plusieurs excipients pharmaceutiquement acceptables.

17. Composition selon la revendication 16, dans laquelle (i) le principe actif est (-)-(R)-2-{4-[[3,4-dihydro-2H-benzopyran-2-yl)méthyl]amino]-butyl}-1,2-benzoisothiazol-3-(2H)-one-1,1-dioxyde ou son sel physiologiquement acceptable et (ii) au moins un médicament anti-Parkinsonien, en combinaison avec un ou plusieurs excipients pharmaceutiquement acceptables.

18. Composition selon la revendication 16 ou 17, dans laquelle l'au moins un médicament anti-Parkinsonien classique est choisi parmi un groupe comprenant L-Dopa (levodopa), L-Dopa combiné avec bensérazide ou carbidopa, des agonistes de dopamine tels que bromocriptine, apomorphine, cabergoline, pramipexol, ropinirol, pergolide, dihydro-α-ergocriptine ou lisuride plus tous les médicaments agissant via une stimulation de récepteurs de dopamine, des inhibiteurs de catéchol-O-méthyle transférase (COMT) tels que entacapone ou tolcapone, des inhibiteurs de monoamine oxydase (MAO) tels que selegiline et des antagonistes de récepteurs de N-méthyl-D-aspartate (NMDA) tels que amantadine ou budipine.

19. Composition selon les revendications 16 à 18, dans laquelle (i) le principe actif est sous la forme de son monohydrochlorure et (ii) le médicament anti-Parkinsonien classique est L-Dopa.
